(19) Europäisches Patentamt
European Patent Office
Office européen des brevets

(11) Numéro de publication: **0 578 726 B1**

(12) # FASCICULE DE BREVET EUROPEEN

(45) Date de publication du fascicule du brevet: **27.12.95**

(51) Int. Cl.⁶: **C12N 7/06**, A61L 2/16, A61K 38/00

(21) Numéro de dépôt: **92908672.6**

(22) Date de dépôt: **27.03.92**

(86) Numéro de dépôt internationale : **PCT/FR92/00276**

(87) Numéro de publication internationale : **WO 92/17570 (15.10.92 92/26)**

(54) **PROCEDE D'INACTIVATION DE VIRUS**

(30) Priorité: **29.03.91 FR 9103892**

(43) Date de publication de la demande: **19.01.94 Bulletin 94/03**

(45) Mention de la délivrance du brevet: **27.12.95 Bulletin 95/52**

(84) Etats contractants désignés: **AT BE CH DE DK ES FR GB GR IT LI LU MC NL SE**

(56) Documents cités:
**EP-A- 0 203 909**
**FR-A- 2 475 572**

(73) Titulaire: **VEGATEC**
**2, place Pablo-Picasso**
**F-94800 Villejuif (FR)**

(72) Inventeur: **SCHMITTHAEUSLER, Roland**
**8, rue du Mont-Cenis**
**F-78180 Montigny-le-Bretonneux (FR)**
Inventeur: **KLAGBA, Wisdom**
**7, rue du Commandant-de-l'Herminier**
**F-75020 Paris (FR)**
Inventeur: **LE GOFFIC, François**
**42, rue Jean-Georget**
**F-92140 Clamart (FR)**

(74) Mandataire: **Stalla-Bourdillon, Bernard**
**Cabinet Nony**
**29, rue Cambacérès**
**F-75008 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

CHEMICAL ABSTRACTS, vol. 111, no. 7, 14 Août 1989, Columbus, Ohio, US; abstract no. 53659P, D. PLUSOUELLEC ET AL.: 'SYNTHESIS AND CHARACTERIZATION OF 6-O-(N-HEPTYLCARBAMOYL)-METHYL-ALPHA-D-GLUCOPYRANOSIDE, A NEW SURFACTANT FOR MEMBRANE STUDIES, p. 383 & ANAL. BIOCHEM., vol. 179, no. 1, 1989, pp. 145-153

CHEMICAL ABSTRACTS, vol. 98, no. 22, 30 Mai 1983, Columbus, Ohio, US; abstract no. 185437N, V.E. BEREZIN ET AL.: 'SEARCH FOR EFFECTIVE PREPARATIONS TO DISINTEGRATE INFLUENZA VIRIONS.' page 361; voir abrégé & IZV. AKAD. NAUK KAZ. SSR, SER. BIOL. no. 1, 1983, pp. 11-15

CHEMICAL ABSTRACTS, vol. 89, no. 3, 17 Juillet 1978, Columbus, Ohio, US; abstract no. 24713Y, H. ARITA ET AL.: 'STUDIES ON ANTIVIRAL GLYCOSIDES. SYNTHESIS AND BIOLOGICAL EVALUATION OF VARIOUS PHENYL GLYCOSIDES.' page 687; voir abrégé & CARBOHYDRATE RESEARCH, vol. 62, no. 1, 1978, pp. 143-154

## Description

La présente invention concerne un procédé d'inactivation de virus dans des compositions contenant des protéines.

Les produits biologiques obtenus notamment par extraction à partir de plasma ou de sang complet peuvent se trouver contaminés par des virus tels que le virus HBV ou les virus HIV. Il est nécessaire dans ces conditions de prévoir une étape d'inactivation de ces virus lors de la préparation de ces produits biologiques.

En outre, ce type de produits contient essentiellement comme principe actif des protéines : facteur de coagulation par exemple, dont l'intégrité doit être préservée. Il importe donc que le procédé d'inactivation ne modifie pas les propriétés desdits produits. Enfin, la technique utilisée ne doit pas non plus générer d'agents susceptibles d'avoir une activité toxique à plus ou moins long terme.

Outre les facteurs de coagulation, d'autres compositions nécessitent une étape d'inactivation, notamment des compositions utilisables comme vaccins ; de même, les produits issus des méthodes de la biologie moléculaire nécessitent une étape d'inactivation virale en cours de purification.

Différentes techniques ont été envisagées pour atteindre cet objectif.

C'est ainsi que le traitement par la chaleur a été préconisé pour l'inactivation virale, par exemple de solution d'immunoglobulines dans EP 177 836. Cependant, ce type de traitement est très délicat étant donné les risques de dénaturation des protéines.

L'inactivation virale peut également être obtenue par traitement de la composition par de la $\beta$ propiolactone, dont l'action est renforçée par des rayonnements U.V. ; EP 268 973 décrit ainsi la préparation de solution d'immunoglobulines G pour injection intraveineuse. Toutefois, on observe également un certain taux de dénaturation des protéines fragiles par ce traitement, en outre, la $\beta$ propiolactone constitue un facteur de risque carcinogénique pour les manipulateurs et risque d'entraîner des formations de néo-antigènes sur les protéines traitées.

On s'est également orienté vers l'utilisation de détergents pour inactiver les virus. L'emploi d'agents tensioactifs pour désinfecter le matériel contaminé par des virus ou des bactéries est connu de longue date.

Ils ont été notamment utilisés dans le but d'éliminer l'infectivité liée aux micro-organismes (US 3962421) ou aux virus (EP-A-0203909, Melnikova et al. VOPR. VIRUSOL. 30, 153-158, 1985), afin d'obtenir des substances vaccinantes sans contagiosité.

Ces deux dernières références citent comme agent tensioactif, l'octyl-$\beta$-D-glucopyranoside, possédant uniquement une partie apolaire et une partie polaire.

Récemment, Cornet et al. dans la publication "Biochemical and Biophysical Research Communications, vol. 167 n° 1, 1990, pages 222-231, ont décrit la solubilisation des protéines d'enveloppe du virus du SIDA, le VIH, par le même tensioactif l'OGP, pour former des virosomes en vue d'élucider les mécanismes moléculaires intervenant dans l'attachement et la fusion des protéines virales avec la cellule hôte.

L'octyl-$\beta$-glucopyranoside est susceptible de péroxidation. De plus, sa méthode de synthèse conduit à un produit où l'on détecte, outre les péroxides, d'autres impuretés mal caractérisées (B.Lorbert et al., Biochimica et Biophysica Acta, 1023 (1990) p 254-265).

L'emploi de l'octyl-$\beta$-D-glucopyranoside, dans l'inactivation virale des préparations à finalité pharmaceutique injectable, n'est donc pas totalement satisfaisant.

La mise en oeuvre de détergents amphiphiles est décrite pour le traitement des protéines plasmatiques dans les brevets US 4 315 919 et 4 314 997.

L'action conjuguée d'un solvant et d'un détergent a été proposée pour l'inactivation virale de protéines du plasma dans le brevet US 4 540 573.

Tous ces brevets font cependant appel à des détergents disponibles sur la marché, préparés de façon industrielle notamment par condensation de polyoxyéthylène et d'acides gras, estérifiés par l'anhydride sorbique.

De telles méthodes de synthèses conduisent à des mélanges complexes de polymères souvent mal définis. Il s'ensuit donc une variation structurale de la composition de tels détergents lorsqu'on passe d'un lot de fabrication à l'autre. D'où le risque d'une reproductibilité aléatoire des expériences dans lesquelles de tels produits sont utilisés.

Enfin, ces détergents ne sont pas inertes vis-à-vis des membranes cellulaires, dont certains composants sont alors extraits préférentiellement, entraînant ainsi un risque de dysfonctionnement des cellules atteintes. Des impuretés très réactives sur les protéines sensibles ont été mises en évidence dans les détergents de type Triton, Tween etc. (Chang, H.W. et al : Anal. Biochem. 104, 112-117 (1980) au C. Brossard et al. : STP Pharma 6, 728-741 (1990).

Le traitement des produits plasmatiques destinés à l'injection chez des patients humains par ces détergents n'est donc pas totalement satisfaisant.

Il est préférable de pouvoir disposer d'une seule molécule pour effectuer de telles inactivations. En effet, une seule molécule est facile à caractériser, sa pureté est connue (supérieure à 98 %) et les impuretés présentes sont également identifiables. Son élimination est facile à vérifier et la reproductibilité de l'expérience biochimique ou biologique est par définition certaine, toutes choses étant égales par ailleurs.

C'est pourquoi la présente invention a pour objet un procédé d'inactivation de virus dans une solution contenant des protéines, caractérisé en ce que :

a) un liposaccharide appartenant à la famille des uréthannes disubstitués de formule générale :

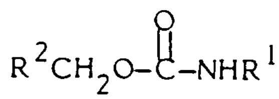

(I)

dans laquelle :

- $R^1$ représente un radical alkyle linéaire comportant n atomes de carbone, n étant un entier compris entre 1 et 20, de préférence valant de 3 à 12, et
- $R^2$ représente un reste d'un aldohexose, éventuellement substitué, est addition né à ladite solution,

b) le liposaccharide est ensuite éliminé de manière à récupérer la solution de protéines purifiée.

Plus particulièrement, la présente invention a pour objet un procédé d'inactivation de virus utilisant un liposaccharide de formule :

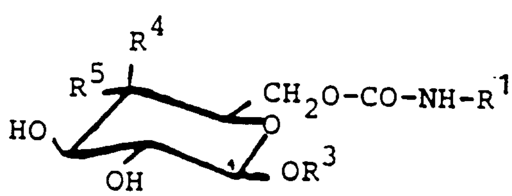

(II)

dans laquelle :

- $R^1$ est un radical alkyle linéaire saturé comportant n atomes de carbone avec n compris entre 1 et 12,
- $R^3$ est H ou $CH_3$,
- $R^4$ et $R^5$ sont indépendamment H ou OH.

Les composés de formule I sont constitués d'un hexose sous forme pyrane qui peut être par exemple le D glucose, le D galactose ou l'$\alpha$ -méthyl D glucoside.

Les parties polaires et apolaires sont reliées par un groupe carbamyl. La partie hydrophobe est constituée par une chaîne alkyle plus ou moins longue.

De manière préférée, dans la formule II, $R^1$ représente un alkyl en C7, $OR^3$ est en position $\alpha$, $R^4$ représente H, $R^5$ représente -OH.

Le procédé de l'invention donne en particulier de bons résultats, quand il est réalisé avec un composé pour lequel, dans la formule II telle qu'elle est définie ci-dessus, $R^3$ est un groupe $CH_3$, c'est-à-dire avec le 6-O-(N-heptylcarbamoyl)méthyl-$\alpha$-D-glucopyranoside, de formule :

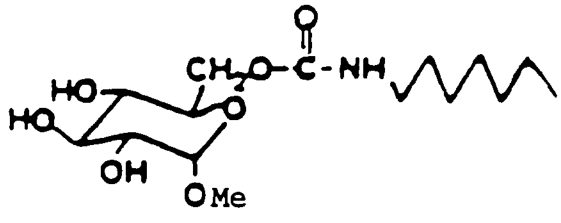

(III)

Ces dérivés proviennent de la condensation de méthylglucose et d'heptanoyle isocyanate.

Leur synthèse est relativement peu coûteuse et peut s'effectuer avec un bon rendement, du fait de la faible formation de produits secondaires lors de la réaction, qui ne conduit pas à des mélanges d'isomères difficilement séparables. Cette synthèse peut notamment être réalisée par le procédé décrit par Plusquellec et al., dans Anal. Biochem., 1989, 179, 145-153).

Une caractéristique de ce liposaccharide est sa facilite d'identification par spectroscopie UV, IR et RMN. Il se présente sous la forme de cristaux blancs. Il a un poids moléculaire égal à 335,6.

Sa concentration micellaire critique est de 19,5 mM, soit 6,5 g/l. Il pourra donc être éliminé facilement par dialyse ou ultra-filtration.

L'analyse thermique différentielle à basse température (-70 °C) jusqu'au point de fusion (108 °-109 °C) montre que ce dérivé ne présente pas de point de recristallisation ou de fusion dans l'intervalle -70 °C à +100 °C, ce qui est un élément favorable pour la congélation et la lyophilisation de solutions contenant (III) contrairement aux dérivés de type Tween 80.

Dans un mode de réalisation préféré du procédé d'inactivation virale selon l'invention, le liposaccharide de formule I, II ou III est ajouté à la solution de protéines, de façon à obtenir une concentration finale comprise entre 1mM et 100mM.

On sait par exemple que le composé de formule III a une solubilité allant jusqu'à 60 g/l dans les tampons usuels à 4 °C.

Le liposaccharide, dans le procédé d'inactivation de virus selon l'invention, peut dans un mode particulier de mise en oeuvre, être additionné à la solution de protéines sous une forme à diffusion lente, par exemple adsorbée sur un support. En particulier, le liposaccharide pourra être adsorbé sur un support constitué de silice. Il pourra être éliminé, après l'inactivation par fixation sur un support particulier ayant une affinité spécifique pour le di-carbamate, le résidu glucose ou le radical heptyl.

L'étude de la cytotoxicité de (III) rapportée à un témoin constitué de cholate de sodium et testé en culture cellulaire de lymphocytes a montré:

- une DC50 à 2,75 mmol/l pour (III), comparé à la DC50 du cholate de sodium = 1,04 mmol/l. Cet essai est évalué par le bleu trypan après 72 heures de contact avec la culture cellulaire, (DC50 : dose cytotoxique 50)
- une DC50 mesurée après stimulation des lymphocytes pour 4 µg de phytohémagglutinine est observée à 0,96 mmol/l pour (III)

et 0,93 mmol/l pour le cholate de Na.

La différence de comportement entre (III) et le cholate de Na pris comme substance de comparaison indique un mécanisme d'action différent de ceux habituellement rencontrés dans les études d'interaction détergent-membrane cellulaire.

Outre, la délipidation de l'enveloppe des virus, on peut invoquer un mécanisme d'action plus complet, qui met en jeu de multiples liaisons entre une molécule de liposaccharide et une molécule de protéine membranaire. Il est utile de signaler la formation de liaisons hydrogènes entre la fonction carbamate et la fonction peptidique de la protéine membranaire.

De préférence, dans le procédé d'inactivation de virus selon l'invention, la température de contact est comprise entre 10 °C et 60 °C.

De manière préférée, le procédé selon l'invention est caractérisé en ce que, après un temps de contact fonction du type de virus à inactiver, et de préférence compris entre 15 minutes et 24 heures, le liposaccharide est retiré de la solution par un solvant non miscible à l'eau ; bien qu'il soit possible de le retirer par d'autres moyens notamment physiques tels que l'ultrafiltration, l'adsorption spécifique, la chromatographie de perméation, par exemple.

Ce solvant non miscible à l'eau peut notamment être choisi dans le groupe comprenant le butanol, l'octanol, le chlorure de méthylène, l'acétate d'éthyle, le chloroforme, l'éther et l'acétate d'amyle.

En particulier, le procédé d'inactivation virale selon l'invention est particulièrement adapte à une solution qui contient des protéines provenant du sang, du plasma, de placenta ou de tout liquide biologique humain ou animal.

Le procédé d'inactivation virale selon l'invention tel qu'il a été défini jusqu'ici peut s'appliquer à une solution de protéines contenant en outre des polypeptides, des glycopeptides, et des glycoprotéines.

Parmi les protéines d'intérêt extraites du sang ou du plasma sanguin, on peut citer notamment le complexe prothrombique, le facteur VIII, le facteur IX de l'hemostase, les immunoglobulines, le fibrinogène, l'hémoglobine, l'interféron.

Le procédé selon l'invention s'appliquera également aux produits issus des techniques de la biologie moléculaire faisant appel à des cellules modifiées pouvant être infectées par des virus.

Enfin, le procédé d'inactivation virale selon l'invention pourra être caractérisé en ce que la solution à traiter contient des protéines ou des polypeptides obtenus par une technique de recombinaison génétique.

La présente invention concerne également des compositions comprenant un liposaccharide de formule I, II, ou III telle que définie précédemment et un véhicule pharmaceutiquement acceptable, utilisables en applications topiques pour le traitement d'affections virales, notamment causées par le virus de l'herpès.

Des essais d'inactivation virale ont été réalisés avec le liposaccharide (III) pour vérifier ses propriétés particulières.

Les exemples suivants sont destinés à illustrer l'invention sans aucunement en limiter la portée.

**EXEMPLE I**

Herpès simplex virus II (HSVII) est entretenu en culture cellulaire et les suspensions virales obtenues ont une dose infectieuse 50% (DI50) à une dilution de $10^{-6}$.

Les suspensions virales sont mises en contact avec 6,5g/l de (III) pendant 1h, 2h, 4h à 30 °C.

Des témoins positifs sont réalisés dans les mêmes conditions d'incubation sans addition de (III).

L'effet virucide de (III) est arrêté par dilution au 1/100e avec du milieu de culture sans virus. La concentration virale atteint alors $10^4$ DI50 pour l'effet de dilution.

**Résultats**

Aucune activité virale de HSVII n'est détectée dans les cellules mises en culture avec la solution traitée par (III) après 1h, 2h, 4h.

La suspension virale témoin présente un titre $10^4$ DI50.

L'inactivation de HSVII est obtenue dans cet essai à hauteur de 4 log minimum.

**EXEMPLE II**

Herpès Simplex Virus II (HSV II) de l'exemple 1 est incubé avec une solution de (III) à 5 g/l en concentration finale dans son milieu de culture.

Des prélèvements sont effectués à intervalles de temps 5, 10, 15, 30 et 60 minutes, puis titrés pour leur contenu en virus infectieux sur cellules réceptrices en culture.

**Résultats**

Aucune activité virale de HSVII n'est détectée après 15 minutes de contact de la suspension virale avec (III) à 5 g/l en concentration finale.

**EXEMPLE III**

Une solution de FVIII purifié contenant 36 U/ml extrait du plasma humain est mis en présence de (III) à différentes concentrations comprises entre 0 et 20 g/l à température ambiante (22 °C). Des prélèvements effectués à 0, 2 h, 4 h, 6 h, et 24 h sont titrés pour évaluer leur teneur en FVIII, sans élimination préalable de (III). Le FVIII purifié est dilué en 1/10e pour l'essai de l'exemple III.

Les résultats figurent dans le tableau A :

| III (g/l) / T(h) | 0 | 3 | 5 | 10 | 20 |
|---|---|---|---|---|---|
| 0 | 3,6 | 4,2 | 4,67 | 4,9 | 4,5 |
| 2 | 3,45 | 4,69 | 4,96 | 4,79 | 4,93 |
| 4 | 4,45 | 5,9 | 5,86 | 3,98 | 3,69 |
| 6 | 3,1 | 4,32 | 4,98 | 4,31 | 3,86 |
| 24 | 1,65 | 3,7 | 2,95 | 2,1 | 1,43 |

Tableau A : FVIII coagulant en U/ml en fonction du temps T de contact avec (III) g/l à 22°C.

Les résultats obtenus montrent la possibilité de doser le FVIII en présence de (III). L'évolution du FVIII à 20 g/l est parallèle à celle du témoin 0 g/l (III) encre 0 et 24 h.

(III) n'empêche pas l'activité du FVIII de baisser au cours du temps de conservation à l'état liquide.

Les différences entre la colonne témoin 0 g/l de III et 20 g/l de (III) ne sont pas significatives.

## EXEMPLE IV

Une solution de FVIII semi-purifié extraite du plasma humain est mise en présence de 0,5 et 10 g/l de (III).

La solution contient 8,2 U/ml de FVIII.

Ce produit est moins purifié que celui de l'exemple III.

Le tableau B résume les résultats obtenus.

| | 0 g/l | 5 g/l | 10 g/l |
|---|---|---|---|
| To | 8,2 (100%) | 6,93 (84,5%) | 5 (72%) |
| To + 20 H | 5,6 (68%) | 3,7 (45%) | n.d.* 0% |

* n.d. = non dosable

Tableau B : stabilité du FVIII en présence de (III) au cours du temps à 22°C (exprimé en U/ml)

Le FVIII semi-purifié perd 55 % de son activité en présence de 5 g/l de (III) en 20 heures de contact à 22 °C.

Par rapport au témoin sans (III), la perte supplémentaire due à la présence de (III) est de 33 %. Ces résultats indiquent que le FVIII en présence de (III) suit une dégradation très voisine de celle du témoin en 20 h de contact.

## EXEMPLE V

Une solution de FVIII semi-purifié est mise en présence de concentrations variables de (III). A titre de référence, on utilise le FVIII semi-purifié traité par un procédé d'inactivation virale à base d'un mélange solvant-détergent. (Tween 80 et tri-h-butylphosphate).

Le second témoin est constitué de la solution de FVIII semi-purifié sans additif.

Le tableau C résume les résultats obtenus :

| (III)g/l / T (h) | Témoin Tween 80 TrinBP | Témoin 0 sans additif | 0,1 | 0,5 | 1 | 2 | 5 |
|---|---|---|---|---|---|---|---|
| 0 | 11,54 (125%) | 9,2 (100%) | 9,1 | 8,77 | 8,6 | 8,74 | 8,46 |
| 20 | 8,62 | 7,16 (77,8%) | 7,63 | 6,92 | 6,51 | 6,47 | 5,95 (65%) |

Tableau C : évolution du FVIII semi-purifié en U:ml en fonction de la concentration de (III) au cours du temps (0 et 20 h).

Par rapport au témoin FVIII sans additifs, la perte supplémentaire entraînée par 5 g/l de (III) après 20 h de contact est de 17 %.

**EXEMPLE VI**

Dans le but d'étudier l'influence de la température, on traite une solution de FVIII semi-purifié par le mélange TnBP/Tween 80 comme témoin et par (III) à raison de 2 g/l à 28 ° C et 37 ° C.

Les résultats obtenus sont résumés dans le tableau D :

| | T en h | 0 | 1 | 2 | 3 | 4 | 5 | 20 |
|---|---|---|---|---|---|---|---|---|
| Temp. = 28°C | Témoin Tween TnBP | 10,23 | 10,71 | 11,94 | n.d. | 9,76 | 9,93 | 9,53 |
| | Témoin 0 g/l de (III) | 7,71 (100%) | 8,74 | 9,56 | n.d. | 8,4 | 8,05 | 8,44 |
| | 2 g/l de (III) | 8,26 | 8,82 | 9,04 | n.d. | 7,62 | 7,45 | 6,11 |
| Temp. = 37°C | Témoin Tween TnBP | 10,23 | 11,25 | 10,96 | 11,8 | 9,74 | 8,17 | 6,82 |
| | Témoin 0 g/l de (III) | 7,71 (100%) | 9,25 | 9,17 | 8,95 | 7,96 | 8,32 | 8,0 |
| | 2 g/l de (III) | 8,26 | 8,77 | 8,6 | 8,62 | 5,18 | 5,95 | 2,57 |

<u>Tableau D</u> : Influence de la température à 28°C et 37°C sur la stabilité du FVIII semi-purifié en présence de 2 g/l de (III) exprimé en U/ml.

Les résultats indiquent que le FVIII semi-purifié conserve son activité en présence de (III) à 2 g/l pendant 3 heures à 37 ° C.

**<u>EXEMPLE VII</u>**

Une solution de FVIII:C est obtenue par clonage dans des cellules d'ovaires de hamster chinois. L'ovaire de hamster chinois (CHO) maintenue en culture continue.

Le FVIII:C exprimé est isolé du milieu de culture et purifié. Il titre 1 U/ml.

Le FVIII:C ainsi purifié est dilué à raison de 4 ml de solution de FVIII:C + 1 ml de solution de (III) à 50 g/l.

On obtient ainsi une solution de FVIII:C contenant 10 g/l de (III) en concentration finale.

Une partie de la solution ainsi constituée est dialysée contre une solution de chlorure de sodium à 9 g/l.

Un témoin est constitué par 4 ml de solution de FVIII:C additionné de 1 ml de solution de chlorure de sodium à 9 g/l.

Après une heure de contact à température 20 ° C, le FVIII:C est dosé dans les trois échantillons.

Les résultats sont résumés dans le tableau E :

| Echantillon | U/ml |
|---|---|
| Témoin F VIII:C | 1,16 |
| F VIII:C avec 10 g/l (III) | 1,3 |
| F VIII:C avec 10 g/l (III) dialysé | 1,1 |

Tableau E : Influence de (III) sur une solution de FVIII:C obtenue par DNA recombinant.

Les résultats obtenus indiquent la bonne stabilité du FVIII:C en présence de (III) à 10 g/l et la possibilité de doser l'activité coagulante de la solution contenant (III) ou ayant été dialysée pour éliminer (III).

**EXEMPLE VIII**

Une protéine de plasma humain présente une activité polyvalente à intérêts thérapeutiques : anti C 1 estérase, anti-kallicréine, anti-plasmine. De plus, cette protéine identifiée comme C 1 Inactivateur (ou inhibiteur de la C 1 estérase) peut également trouver sa place dans la thérapeutique substitutive (déficit congénital se traduisant par un neurangiome).

Un procédé a été mis au point pour extraire et purifier le C 1 Inactivateur à partir du plasma humain. Cependant, la protéine ainsi purifiée est difficile à pasteuriser 10 h à 60 °C, sans recours à des stabilisants.

La protéine a donc été traitée d'une part avec le procédé solvant-détergent, d'autre part avec (III) à une concentration finale de 6,5 g/l pendant 6 h à 24 °C, dans le but d'obtenir l'inactivation des virus éventuellement présents dans la matière première.

Les résultats obtenus figurent dans les tableaux F et G, correspondant à deux essais de purification incluant une étape d'inactivation virale.

| Nature du Traitement | Essai témoin sans additifs | | | Essai Tween-TnBP 1% + 0,3% | | | Essai (III) à 6,5 g/l | | |
|---|---|---|---|---|---|---|---|---|---|
| Temps d'incubation | 6 h - 24°C | | | 6 h - 24°C | | | 6 h - 24°C | | |
| | Cl ina mg | Rdt % | Pureté | Cl ina mg | Rdt % | Pureté | Cl ina mg | Rdt % | Pureté |
| Prééluat de PPSB | 716 | 100 | 6,2 | 645 | 100 | 5,1 | 652 | 100 | 5,3 |
| DEAE sépharose éluat | 670 | 93,5 | 22,5 | 604 | 93,6 | 13,7 | 682 | 100 | 17,7 |
| Précipité PEG 30% après reprise | 630 | 87,9 | 41,4 | 480 | 74,4 | 31,7 | 540 | 82,8 | 32,7 |
| S-Sépharose éluat | 170 | 23,7 | >98 | 123 | 19,1 | >98 | 137 | 21 | >98 |

Tableau F : Influence du procédé d'inactivation virale sur la purification de C 1 Inactivateur : Essai n° 1

| Nature du Traitement | Essai témoin sans additifs | | | Essai Tween-TnBP 1% + 0,3% | | | Essai (III) à 6,5 g/l | | |
|---|---|---|---|---|---|---|---|---|---|
| Temps d'incubation | 6 h - 24°C | | | 6 h - 24°C | | | 6 h - 24°C | | |
| | Cl ina mg | Rdt % | Pureté | Cl ina mg | Rdt % | Pureté | Cl ina mg | Rdt % | Pureté |
| Prééluat de PPSB | 731 | 100 | 5,3 | 684 | 100 | 3,7 | 721 | 100 | 4,4 |
| DEAE sépharose éluat | 594 | 81,2 | 15,4 | 607 | 88,7 | 18,7 | 522 | 72,3 | 10,7 |
| Précipité PEG 30% après reprise | 421 | 57,5 | 20,4 | 338 | 49,4 | 18,2 | 367 | 50,9 | 23,6 |
| S-Sépharose éluat | 190 | 26 | >98 | 125 | 18,3 | >98 | 114 | 15,8 | >98 |

Tableau G : Influence du procédé d'inactivation virale sur la purification de C 1 Inactivateur : Essai n° 2

Les deux essais effectués montrent que le produit (III) ajouté en tant qu'inactivateur de virus ne compromet pas le rendement de purification du C 1 Inactivateur ni la pureté finale de la protéine.

Le tableau H montre l'influence du traitment d'inactivation virale sur les propriétés inhibitrices du C 1 Ina essayé sur trois enzymes : C 1 estérase, kallicréine, plasmine.

Le témoin est incubé 6 h à 24 ° C mais ne contient aucun additif inactivant.

| | Essai n° | Témoin 6 h - 24°C (%) | Tween-TnBP 6 h - 24°C (%) | (III) à 6,5 g/l 6 h - 24°C (%) |
|---|---|---|---|---|
| Activité anti C 1 estérase | 1 | 98,5 | 95,5 | 96,2 |
| | 2 | 95,7 | 97,0 | 100 |
| Activité anti-kallicréine | 1 | 95,3 | 85,4 | 95,3 |
| | 2 | 86,3 | 90,1 | 94,1 |
| Activité anti-plasmine | 1 | 95,8 | 88,6 | 80,2 |
| | 2 | 82,3 | 90,6 | 96,6 |

Tableau H : Influence du traitement d'inactivation virale sur la capacité inhibitrice du C 1 Inactivateur vis-à-vis de trois enzymes.

L'activité inhibitrice est exprimée en % de l'activité inhibitrice du C 1 Inactivateur purifié sans additif et dosé à To.

**EXEMPLE IX**

Dans le but d'extraire (III) d'un milieu aqueux, il est possible d'utiliser des techniques de dialyse, d'ultrafiltration de chromatographie de perméation, de chromatographie d'interactions hydrophobes, de chromatographie d'échange d'ions.

Une autre possibilité est d'extraire (III) par un système à deux phases non miscibles l'une aqueuse (A) l'autre organique (o). Le coefficient de partage de (III) entre les deux phases permet l'extraction sélective de (III) du milieu aqueux, selon le solvant (o) choisi.

Le tableau J montre l'efficacité d'extraction de (III) à partir d'une solution aqueuse (A) contenant 6,5 g/l de (III) et 9 g/l de NaCl. L'extraction s'effectue en mettant en contact 50 ml de solvant (o) et 100 ml de solution aqueuse (A).

| Solvant | % extrait (III) dans O |
|---|---|
| Acétate d'éthyle | 68 |
| Chlorure de méthylène | 63 |
| Chloroforme | 77 |
| Trichloro 1,1,2 éthane | 0 |
| 1-octanol | 95 |

**Tableau J** : extraction de (III) par solvants non miscibles à l'eau.

## EXEMPLE X

Dans le but d'étudier l'efficacité du traitement de plasma par le liposaccharide (I), on a inoculé 10 ml de plasma frais avec une suspension de virus Herpès Aujeszky pour obtenir un titre final de 9 x $10^6$ UFP/ml (titre théorique).

Le mélange est alors additionné du liposaccharide(I) à raison de 6,5 g/l en concentration finale et incubé à 30° C ± 1° C.

Des prélèvements sont effectués à intervalle de temps réguliers entre 5 minutes et 1 heures en vue de déterminer la cinétique d'inactivation du virus Aujeszky en milieu plasmatique.

La mise en culture des prélèvements sur cellules Vero permet de déterminer les titres de virus résiduel par la méthode des plages.

Des contrôles positifs et négatifs sont effectués dans la même expérience.

Le facteur de réduction calculé est supérieur à 4,44 mg en 5 minutes de traitement.

Les résultats de l'essai figurent dans le tableau K suivant :

| ECHANTILLONS | PSEUDORAGE PORCINE* Titre infectieux | | FACTEUR DE REDUCTION ** |
|---|---|---|---|
| | UFP/ml | log UFP/ml | |
| Produit de départ non infecté | non toxique au 1/40 | | |
| Virus stock utilisé pour l'expertise, dilué dans le milieu de culture, congelé à -75 ± 5°C | $2{,}91.\ 10^{8}$ | | |
| Virus stock utilisé pour l'expérience, dilué dans le milieu de culture laissé 1 heure à 30°C puis congelé à -75 ± 5°C | $7{,}06.\ 10^{7}$ | | |
| Virus dilué dans le produit de départ et congelé à -75 ± 5°C | $2{,}76.\ 10^{6}$ | 6,44 | |
| Virus dilué dans le produit de départ laissé 1 heure à 30°C puis congelé à -75 ± 5°C | $2{,}7.\ 10^{4}$ | 4,43 | 2,01 |

Tableau K : Etude de l'inactivation du virus de la pseudorage porcine (virus herpes d'aujeszky) au cours du traitement par liposaccharide hecameg à 30° C

| | | | |
|---|---|---|---|
| Titre du virus dilué dans le produit de départ, traité par 6,5 g/litre d'HECAMEG et incubé à 30°C pendant : | | | |
| 5 minutes | < 100 | < 2 | > 4,44 |
| 10 minutes | < 100 | < 2 | > 4,44 |
| 15 minutes | < 100 | < 2 | > 4,44 |
| 30 minutes | < 100 | < 2 | > 4,44 |
| 1 heure | < 100 | < 2 | > 4,44 |

* Les titres sont exprimés en Unités Formant Plages par millilitre (UFP/ml). Ils tiennent compte de la limite de toxicité du produit sur les cellules utilisées pour le titrage.

** Les facteurs de réduction sont exprimés en log UFP. Ceux-ci sont calculés en tenant compte de la chute de titre due à la durée de l'expérience.

Tableau K ( Suite )

## Revendications

1. Procédé d'inactivation de virus dans une solution contenant des protéines, caractérisé en ce que :

   a) un liposaccharide appartenant à la famille des uréthannes disubstitués de formule générale :

   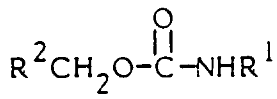 (I)

   dans laquelle :

   - $R^1$ représente un radical alkyle saturé comportant n atomes de carbone, n étant un entier compris entra 1 et 20, de préférence 3 à 12
   - $R^2$ représente un reste d'un aldohexose, éventuellement substitué, est additionné à ladite solution,

   b) après un temps de contact, le liposaccharide est éliminé de manière à récupérer la solution de protéines purifiée.

2. Procédé d'inactivation de virus selon la revendication 1, caractérisé en ce que le liposaccharide a pour formule :

   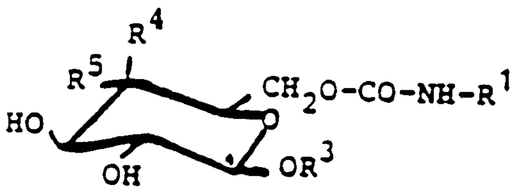 (II)

   dans laquelle :

   - $R^1$ est un radical alkyle linéaire saturé comportant n atomes de carbone avec n compris entre 1 et 12,
   - $R^3$ est H ou $CH_3$,
   - $R^4$ et $R^5$ sont indépendamment H ou OH.

3. Procédé d'inactivation de virus selon l'une des revendications 1 et 2, caractérisé en ce que n = 7.

4. Procédé d'inactivation de virus selon l'une des revendications 1 à 3, caractérisé en ce que le liposaccharide est le 6-O-(N-heptylcarbamoyl)methyl-α-D glucopyranoside.

5. Procédé d'inactivation de virus selon l'une des revendications 1 à 4, caractérisé en ce que le liposaccharide est additionné à la solution de protéines, de façon à obtenir une concentration finale comprise entre 1 mM et 100mM.

6. Procédé d'inactivation de virus selon l'une des revendications 1 à 5, caractérisé en ce que le liposaccharide est additionné à la solution de protéines sous une forme à diffusion lente.

7. Procédé d'inactivation de virus selon l'une des revendications 1 à 6, caractérisé en ce que la température de contact est comprise entre 10 ° C et 60 ° C.

8. Procédé d'inactivation de virus selon l'une des revendications 1 à 7, caractérisé en ce que le liposaccharide est retiré de la solution après un temps de contact compris entre 15 minutes et 24 heures.

**9.** Procédé d'inactivation de virus selon l'une des revendications 1 à 8, caractérisé en ce que le liposaccharide est retiré de la solution après un temps de contact, par un solvant non miscible à l'eau.

**10.** Procédé d'inactivation de virus selon la revendication 9, caractérisé en ce que le solvant non miscible à l'eau est choisi dans le groupe comprenant notamment le butanol, l'octanol, le chlorure de méthylène, l'acétate d'éthyle, le chloroforme, l'éther et l'acétate d'amyle.

**11.** Procédé d'inactivation de virus selon l'une des revendications 1 à 8, caractérisé en ce que le liposaccharide est retiré de la solution après un temps de contact par adsorption sur un support ayant une affinité spécifique pour le dicarbamate.

**12.** Procédé d'inactivation de virus selon l'une des revendications 1 à 11, caractérisé en ce que la solution contient des protéines provenant du sang, du plasma, de placenta, ou de liquides biologiques, humain ou animal.

**13.** Procédé d'inactivation de virus selon la revendication 12, caractérisé en ce que la solution contient également des polypeptides, des glycopeptides, et des glycoprotéines.

**14.** Procédé d'inactivation de virus selon l'une des revendications 1 à 11, caractérisé en ce que la solution à traiter contient des protéines ou des polypeptides obtenus par une technique de recombinaison génétique.

## Claims

**1.** Method for the inactivation of virus in a solution containing proteins, characterized in that:

a) a liposaccharide belonging to the family of disubstituted urethanes of general formula:

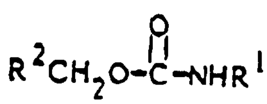

(I)

in which:

- $R^1$ represents a saturated alkyl radical containing n carbon atoms, n being an integer between 1 and 20, preferably 3 to 12
- $R^2$ represents a residue of an aldohexose, optionally substituted,

is added to the said solution,

b) after a contact time, the liposaccharide is removed so as to recover the purified protein solution.

**2.** Virus inactivation method according to Claim 1, characterized in that the liposaccharide is of the formula:

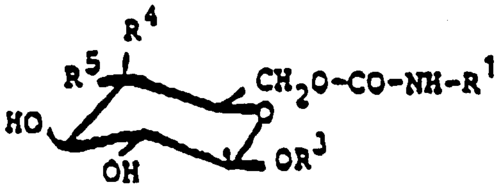

(II)

in which:

- $R^1$ is a saturated linear alkyl radical containing n carbon atoms, with n between 1 and 12,
- $R^3$ is H or $CH_3$,
- $R^4$ and $R^5$ are, independently, H or OH.

**3.** Virus inactivation method according to either of Claims 1 and 2, characterized in that n = 7.

4. Virus inactivation method according to one of Claims 1 to 3, characterized in that the liposaccharide is 6-O-(N-heptylcarbamoyl)methyl-α-D-glucopyranoside.

5. Virus inactivation method according to one of Claims 1 to 4, characterized in that the liposaccharide is added to the protein solution so as to obtain a final concentration of between 1mM and 100mM.

6. Virus inactivation method according to one of Claims 1 to 5, characterized in that the liposaccharide is added to the protein solution in a slow-diffusion form.

7. Virus inactivation method according to one of Claims 1 to 6, characterized in that the contact temperature is between 10 °C and 60 °C.

8. Virus inactivation method according to one of Claims 1 to 7, characterized in that the liposaccharide is removed from the solution after a contact time of between 15 minutes and 24 hours.

9. Virus inactivation method according to one of Claims 1 to 8, characterized in that the liposaccharide is removed from the solution, after a contact time, with a water-immiscible solvent.

10. Virus inactivation method according to Claim 9, characterized in that the water-immiscible solvent is chosen from the group comprising, in particular, butanol, octanol, methylene chloride, ethyl acetate, chloroform, ether and amyl acetate.

11. Virus inactivation method according to one of Claims 1 to 8, characterized in that the liposaccharide is removed from the solution, after a contact time, by adsorption on a support having a specific affinity for dicarbamate.

12. Virus inactivation method according to one of Claims 1 to 11, characterized in that the solution contains proteins originating from blood, from plasma, from placenta or from biological fluids, human or animal.

13. Virus inactivation method according to Claim 12, characterized in that the solution also contains polypeptides, glycopeptides and glycoproteins.

14. Virus inactivation method according to one of Claims 1 to 11, characterized in that the solution to be treated contains proteins or polypeptides obtained by a genetic recombination technique.

**Patentansprüche**

1. Verfahren zur Virusinaktivierung in einer proteinhaltigen Lösung, dadurch gekennzeichnet, daß man
   a) ein Liposaccharid aus der Familie der di-substituierten Urethane der allgemeinen Formel:

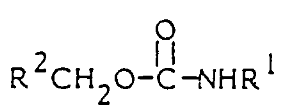 (I)

   in der:
   - $R^1$ einen gesättigten Alkylrest mit n Kohlenstoffatomen bedeutet, wobei n eine ganze Zahl zwischen 1 und 20, Vorzugsweise 3 bis 12 bedeutet,
   - $R^2$ einen gegebenenfalls substituierten Rest einer Aldohexose bedeutet, der Lösung zugibt und

   b) nach einer Kontaktzeit das Liposaccharid entfernt, so daß die gereinigte Lösung der Proteine wiedergewonnen wird.

2. Verfahren zur Virusinaktivierung nach Anspruch 1, dadurch gekennzeichnet, daß das Liposaccharid die Formel:

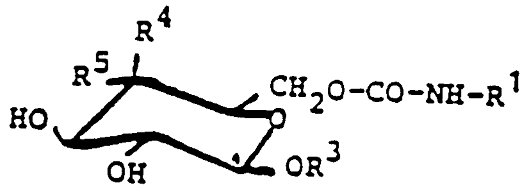

(II)

aufweist, in der

- $R^1$ ein lineares, gesättigtes Alkylradikal mit n Kohlenstoffatomen ist,

wobei n zwischen 1 und 12 bedeutet,

- $R^3$ die Bedeutung von Wasserstoff oder $CH_3$ hat und
- $R^4$ und $R^5$ unabhängig voneinander H oder OH bedeuten.

**3.** Verfahren zur Virusinaktivierung nach einem der Ansprüche 1 und 2, dadurch gekennzeichnet, daß n = 7 ist.

**4.** Verfahren zur Virusinaktivierung nach einem der Ansprüche 1 bis 3, dadurch gekennzeichnet, daß das Liposaccharid 6-O-(N-Heptylcarbamoyl)methyl-α-D Glucopyranosid ist.

**5.** Verfahren zur Virusinaktivierung nach den Ansprüchen 1 bis 4, dadurch gekennzeichnet, daß das Liposaccharid der Proteinlösung auf eine Weise zugegeben wird, daß man eine Endkonzentration zwischen 1 mM und 100 mM erhält.

**6.** Verfahren zur Virusinaktivierung nach einem der Ansprüche 1 bis 5, dadurch gekennzeichnet, daß das Liposaccharid der Lösung der Proteine mittels langsamer Diffusion zugegeben wird.

**7.** Verfahren zur Virusinaktivierung nach einem der Ansprüche 1 bis 6, dadurch gekennzeichnet, daß die Kontakttemperatur zwischen 10 °C und 60 °C liegt.

**8.** Verfahren zur Virusinaktivierung nach einem der Ansprüche 1 bis 7, dadurch gekennzeichnet, daß das Liposaccharid aus der Lösung nach einer Kontaktzeit zwischen 15 Min. und 24 Std. entnommen wird.

**9.** Verfahren zur Virusinaktivierung nach einem der Ansprüche 1 bis 8, dadurch gekennzeichnet, daß das Liposaccharid aus der Lösung nach der Kontaktzeit durch ein mit Wasser nicht-mischbares Lösungsmittel entzogen wird.

**10.** Verfahren zur Virusinaktivierung nach Anspruch 9, dadurch gekennzeichnet, daß das mit Wasser nichtmischbare Lösungsmittel aus der Gruppe enthaltend im besonderen Butanol, Octanol, Methylenchlorid, Ethylacetat, Chloroform, Ether und Amylacetat ausgewählt wird.

**11.** Verfahren zur Virusinaktivierung nach einem der Ansprüche 1 bis 8, dadurch gekennzeichnet, daß das Liposaccharid aus der Lösung nach der Kontaktzeit durch Adsorption auf einen Träger entzogen wird, der für das Dicarbamat eine spezifische Affinität aufweist.

**12.** Verfahren zur Virusinaktivierung nach einem der Ansprüche 1 bis 11, dadurch gekennzeichnet, daß die proteinhaltige Lösung aus Blut, Plasma, Plazenta oder humanen oder tierischen, biologischen Flüssigkeiten stammt.

**13.** Verfahren zur Virusinaktivierung nach Anspruch 12, dadurch gekennzeichnet, daß die Lösung auch Polypeptide, Glycopeptide und Glycoproteine enthält.

**14.** Verfahren zur Virusinaktivierung nach einem der Ansprüche 1 bis 11, dadurch gekennzeichnet, daß die zu behandelnde Lösung Proteine oder Polypeptide enthält, die durch rekombinante Gentechnik erhalten wurde.